# EUROPEAN PATENT SPECIFICATION

(11) **EP 2 747 745 B1**
(45) Date of publication and mention of the grant of the patent: **02.11.2016**
(21) Application number: 12819726.6
(22) Date of filing: 13.07.2012
(51) Int. Cl.: A61K 8/34, A61K 8/30, A61K 8/92, A61Q 15/00

(54) **SOFT SOLID ANTIPERSPIRANT COMPOSITIONS INCLUDING STEARYL ALCOHOL AND CETYL AND/OR MYRISTYL ALCOHOL**
SCHWEISSHEMMENDE WEICHE FESTSTOFFZUSAMMENSETZUNGEN MIT STEARYLALKOHOL UND CETYL- UND/ODER MYRISTYLALKOHOL
COMPOSITIONS SOLIDES MOLLES D'ANTI-TRANSPIRANT CONTENANT DE L'ALCOOL STÉARYLIQUE ET DE L'ALCOOL CÉTYLIQUE ET/OU MYRISTYLIQUE

(30) Priority: 29.07.2011 US 201113193892
(43) Date of publication of application: 02.07.2014
(73) Proprietor: The Dial Corporation, Scottsdale, AZ 85255 (US)
(72) Inventor: YARLAGADDA, Travis T., Phoenix, Arizona 85024 (US)
(74) Representative: Henkel IP Department
(86) International application number: PCT/US2012/046614
(87) International publication number: WO 2013/019383

(56) References cited:
- WO-A1-2007/082063
- WO-A2-2012/006392
- WO-A2-2012/006392
- CA-C- 2 142 577
- US-A- 4 202 879
- US-A- 4 822 603
- US-A- 4 944 937
- US-A- 5 968 490
- US-A1- 2005 095 210
- US-B1- 6 428 777

## Description

### FIELD OF THE INVENTION

The present invention generally relates to antiperspirant compositions, and more particularly relates to soft solid antiperspirant compositions with improved glide and texture.

### BACKGROUND OF THE INVENTION

Antiperspirants and deodorants are popular personal care products used to prevent or eliminate perspiration and body odor caused by perspiration. Antiperspirants typically prevent the secretion of perspiration by blocking or plugging sweat-secreting glands, such as those located at the underarms. Deodorants counteract or mask the unwanted odors caused by bacterial flora in secreted perspiration.

CA 2142577A1 discloses water-containing gelled or stick antiperspirants, which do not comprise waxes, but have sufficient structural integrity to perform as a gelled or stick deodorant. US 4202879 A and US 6428777B1 deal with sticks that are dimensionally stable.

Soft solid antiperspirants, also described as creams or gels, are increasingly desired by the population due to their inclusion of active antiperspirant compounds that block or prevent the secretion of perspiration and its accompanying odors, and to their ease of application. A soft solid product conventionally comprises the antiperspirant composition in a container, which is usually in the form of a canister or barrel. The canister is provided at one end with a closure perforated by one or more apertures and at the opposite end with a piston for urging the canister contents through the apertures. Such compositions flow readily when subjected to pressure, but when the pressure is released they cease to flow and generally do not leak. Accordingly, for application, pressure is applied to extrude an amount of the antiperspirant composition through the apertures, then pressure is relieved. The dispensed amount of antiperspirant is then distributed topically by rubbing the perforated closure across the chosen area of body skin, such as around the underarm.

Soft solid antiperspirant compositions often include silicone in order to provide the proper consistency. Cyclic silicones such as cyclopentasiloxane and tetrasiloxane have drawn the attention of environment concerns, but remain ubiquitous in invisible solid antiperspirants. Alternative carrier fluids may consist of alkanes and/or isoparaffins. Further antiperspirant compositions often comprise stearyl alcohol as a desired structurant that imparts the antiperspirant with some hardness so that it can be stored within and controllably dispensed from the canister without leaking. However, while, not wishing to be bound by theory, it is believed that, after manufacture, the stearyl alcohol may be responsible for visible crystals that form on the invisible solid antiperspirants. These crystals can cause a visibly inhomogeneous and unpleasant surface. Further, it may lead to an undesired glide feel or texture to some users during application of the antiperspirant.

Accordingly, it is desirable to provide soft solid antiperspirant compositions that have improved glide and texture during application. In addition, it is desirable to provide soft solid antiperspirant compositions without silicone. Furthermore, other desirable features and characteristics of the present invention will become apparent from the subsequent detailed description of the invention and the appended claims, taken in conjunction with the accompanying drawings and this background of the invention.

### BRIEF SUMMARY OF THE INVENTION

In accordance with a first embodiment of the invention, a soft solid antiperspirant composition comprises an active antiperspirant compound present in an amount of from 18.0 to 25.0 weight percent of the soft solid antiperspirant composition, a first fatty alcohol constituent comprising stearyl alcohol present in an amount of from 5.0 to 25.0 weight percent of the soft solid antiperspirant composition, a second fatty alcohol constituent comprising C₁₄ to C₁₆ fatty alcohol present in an amount of 2.5 to 12.0 weight percent of the soft solid antiperspirant composition, and wax having a melting point of at least 70°C and present in an amount of from 3.0 to 10.0 weight percent of the soft solid antiperspirant composition, and wherein the container is in the form of a canister 12, which includes
- a top covering 14 that is formed with a plurality of apertures 16 and
- a dial 18 threadably connected to a piston head 20, which is selectively raised, as the dial 18 is rotated,
by which the volume 22 between the piston head 20 and the top covering 14 is reduced and pressure is applied to the soft solid antiperspirant composition 10, resulting in the extrusion of a desired amount of the soft solid antiperspirant composition 10 from the canister 12 through the apertures 16.

### BRIEF DESCRIPTION OF THE DRAWINGS

The present invention will hereinafter be described in conjunction with the following drawing figure wherein:

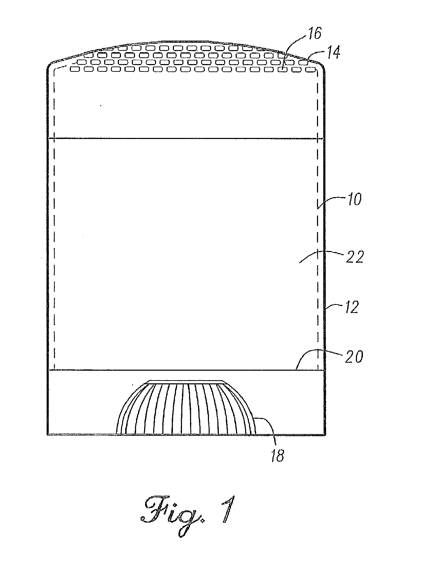

FIG. 1 is a front view of a soft solid antiperspirant composition in accordance with an exemplary embodiment.

### DETAILED DESCRIPTION OF THE INVENTION

The various embodiments contemplated herein relate to a soft solid antiperspirant composition that comprises stearyl alcohol as a desired structurant. In this regard, the various embodiments comprise cetyl alcohol, myristyl alcohol, or a combination thereof. Further, the various embodiments contemplated herein achieve soft solid consistency without the use of silicone. The following soft solid formulations result in improved spreadability on skin, low residue applications, and high efficacy. The embodiments exhibit a consumer preferred texture during application.

In an embodiment shown in FIG. 1, the soft solid antiperspirant composition 10 contemplated herein is packaged in a canister 12 for application. As shown, the canister 12 includes a top covering 14 that is formed with a plurality of apertures 16. Further, the canister 12 includes a dial 18 threadably connected to a piston head 20. As the dial 18 is rotated, the piston head 20 is selectively raised. Accordingly, the volume 22 between the piston head 20 and the top covering 14 may be reduced, and pressure applied to the soft solid antiperspirant composition 10. As a result of the selectively applied pressure, a desired amount of the soft solid antiperspirant composition 10 is extruded from the canister 12 through the apertures 16. Then, the extruded amount of soft solid antiperspirant composition 10 is rubbed against a skin surface.

The various embodiments of the soft solid antiperspirant composition 10 contemplated herein comprise a water-soluble active antiperspirant compound. Active antiperspirant compounds contain at least one active ingredient, typically metal salts, that are thought to reduce perspiration by diffusing through the sweat ducts of apocrine glands (sweat glands responsible for body odor) and hydrolyzing in the sweat ducts, where they combine with proteins to form an amorphous metal hydroxide agglomerate, plugging the sweat ducts so perspiration cannot diffuse to the skin surface. Some active antiperspirant compounds that may be used in the first and/or second portions include astringent metallic salts, especially inorganic and organic salts of aluminum, zirconium, and zinc, as well as mixtures thereof. Particularly preferred are aluminum-containing and/or zirconium-containing salts or materials, such as aluminum halides, aluminum chlorohydrates, aluminum hydroxyhalides, zirconyl oxyhalides, zirconyl hydroxyhalides, and mixtures thereof. Exemplary aluminum salts include those having the general formula Al₂(OH)ₐCl_{b} x (H₂O), wherein a is from 2 to about 5; the sum of a and b is about 6; x is from about 1 to about 6; and wherein a, b, and x may have non-integer values. Exemplary zirconium salts include those having the general formula ZrO(OH)₂₋ₐClₐ x (H₂O), wherein a is from about 1.5 to about 1.87, x is from about 1 to about 7, and wherein a and x may both have non-integer values. Particularly preferred zirconium salts are those complexes that additionally contain aluminum and glycine, commonly known as ZAG complexes.

These ZAG complexes contain aluminum chlorohydroxide and zirconyl hydroxy chloride conforming to the above-described formulas. Examples of active antiperspirant compounds suitable for use in the various embodiments contemplated herein include aluminum dichlorohydrate, aluminum-zirconium octachlorohydrate, aluminum sesquichlorohydrate, aluminum chlorohydrex propylene glycol complex, aluminum dichlorohydrex propylene glycol complex, aluminum sesquichlorohydrex propylene glycol complex, aluminum chlorohydrex polyethylene glycol complex, aluminum dichlorohydrex polyethylene glycol complex, aluminum sesquichlorohydrex polyethylene glycol complex, aluminum-zirconium trichlorohydrate, aluminum zirconium tetrachlorohydrate, aluminum zirconium pentachlorohydrate, aluminum zirconium octachlorohydrate, aluminum zirconium trichlorohydrex glycine complex, aluminum zirconium tetrachlorohydrex glycine complex, aluminum zirconium pentachlorohydrex glycine complex, aluminum zirconium octachlorohydrex glycine complex, zirconium chlorohydrate, aluminum chloride, aluminum sulfate buffered, and the like, and mixtures thereof.

In a preferred embodiment, the antiperspirant compound is aluminum zirconium pentachlorohydrex. The antiperspirant composition according to the invention comprises an active antiperspirant compound present in an amount of from 18.0 to 25.0 weight percent of the soft solid antiperspirant composition.

Further included in the antiperspirant composition is a first fatty alcohol, comprising stearyl alcohol, wherein the first fatty alcohol is present in an amount of from 5.0 to 25.0 weight percent of the soft solid antiperspirant composition. Stearyl alcohol serves as a structurant that provides, at least in part, the soft solid consistency of the antiperspirant composition.

The various embodiments of the antiperspirant composition 10 contemplated herein further comprise a second fatty alcohol, such as cetyl alcohol (comprising 16 carbons), myristyl alcohol (comprising 14 carbons), or a combination thereof (hereinafter, referred to collectively as a "a C₁₄ to C₁₆ fatty alcohol". Based on information and belief, the C₁₄ to C₁₆ fatty alcohols likely form a fatty alcohol matrix with stearyl alcohol that prevents the formation of crystals on the antiperspirant composition upon manufacture thereof. The second fatty alcohol constituent comprising C₁₄ to C₁₆ fatty alcohol is present in an amount of 2.5 to 12.0 weight percent of the soft solid antiperspirant composition. In another exemplary embodiment, the C₁₄ to C₁₆ fatty alcohol and the stearyl alcohol are present in a C₁₄ to C₁₆ fatty alcohol:stearyl alcohol ratio of from about 1:2 to about 1:6.

In some embodiments, because of the low melting point of the C₁₄ to C₁₆ fatty alcohol (about 49°C for cetyl alcohol and about 37-39°C for myristyl alcohol) compared to stearyl alcohol (about 60°C), the addition of the C₁₄ to C₁₆ fatty alcohol may cause the antiperspirant composition to have a lower than desired melting point. Because it is desirable for the antiperspirant composition to be thermally stable and maintain a soft solid form at 45°C, the composition comprises a wax having a melting point of at least 70°C, wherein the wax is present in an amount of from 3.0 to 10.0 weight percent of the soft solid antiperspirant composition. For example, castor wax, or hydrogenated castor oil, may be included in the antiperspirant composition in an amount in the range of from 2.8 or 3.0 to 7.0 or 10.0 wt. %, preferably from 4 to 6 wt. % of the antiperspirant compound. Alternatively or additionally, it may be desirable to use a polyethylene wax as wax having a melting point of at least 80°C. The polyethylene wax, such as high molecular weight (high MW) polyethylene, can add thermal stability to the antiperspirant compound. As used herein, the term "high molecular weight polyethylene" or "high MW polyethylene" means polyethylene having a molecular weight of from about 200 to about 5000 Daltons (Da). High MW polyethylene has a melting point of about 70°C - 100°C and can raise the melting point of the antiperspirant composition. In one embodiment, the antiperspirant composition comprises from 0 or 1.0 to 2.0 or 10.0 wt. % high MW polyethylene. In a preferred embodiment, the high MW polyethylene has a molecular weight of about 500 Da.

Other structurants and/or gellants (hereinafter referred to collectively as "structurants") that, along with stearyl alcohol, can facilitate the soft solid consistency of the antiperspirant composition include naturally-occurring or synthetic waxy materials or combinations thereof. Suitable structurants, including waxes and gellants, are often selected from fatty alcohols containing from 12 to 30 carbons, such as behenyl alcohol and sterols such as lanosterol. As used herein, the term "fatty" means a long chain aliphatic group, such as at least 8 or 12 linear carbons, which is frequently linear, not branched, and is typically saturated, but which can alternatively be branched and/or unsaturated. It is possible for the fatty acid to contain a hydroxyl group, as in 12-hydroxystearic acid, for example as part of a gellant combination, and to employ amido or ester derivates thereof.

Other structurants can comprise hydrocarbon waxes such as paraffin waxes, microcrystalline waxes, ceresin, squalene, and polyethylene waxes. Other suitable structurants are waxes derived or obtained from plants or animals such as hydrogenated soybean oil, carnauba, spermacetti, candelilla, beeswax, modified beeswaxes, and Montan wax and individual waxy components thereof. It is also suitable herein to employ a mixture of wax structurants. Suitable mixtures of structurants can reduce the visibility of active antiperspirant compounds deposited on the skin and result in either a soft solid or a firm solid.

The antiperspirant compositions also may comprise a high refractive index (R.I.) hydrophobic compound. As used herein, the term "high refractive index" means a refractive index of no less than about 1.4. The high R.I. hydrophobic compound also facilitates the minimization and/or prevention of a white residue on the skin by masking the active antiperspirant salt that stays upon the skin upon evaporation of the carrier, discussed in more detail below. Examples of high R.I. hydrophobic compounds for use in the antiperspirant products include PPG-14 butyl ether, C₁₂-C₁₅ alkyl benzoate, such as Finsolv TN® available from Innospec of the United Kingdom, and phenyl dimethicone. In a preferred embodiment, the antiperspirant composition comprises PPG-14 butyl ether. In another preferred embodiment, the antiperspirant composition comprises from 5.0 to 15.0 wt. % high R.I. hydrophobic compound.

In addition to the ingredients identified above, the antiperspirant composition may comprise additives, such as those used in conventional antiperspirants. These additives include, but are not limited to, parfum or fragrances, including encapsulated fragrances, dyes, pigments, preservatives, antioxidants, moisturizers, and the like. These optional ingredients can be included in the antiperspirant composition in an amount of 0 to 20 wt. %. In a preferred embodiment, the antiperspirant composition comprises from 1.0 to 8.0 wt. % myristyl myristate, which provides a conditioning effect to the skin. In another preferred embodiment, the antiperspirant composition comprises from 0.1 to 3.0 wt.% fragrance.

The antiperspirant composition further comprises at least one hydrophobic carrier. An example of suitable hydrophobic carriers includes liquid siloxanes and particularly volatile polyorganosiloxanes, that is, liquid materials having a measurable vapor pressure at ambient conditions. The polyorganosiloxanes can be linear or cyclic or mixtures thereof. The linear volatile silicones generally have viscosities of less than about 5 centistokes at 25°C, while the cyclic volatile silicones have viscosities under 10 centistokes. Preferred siloxanes include cyclomethicones, which have from about 3 to about 6 silicon atoms, such as cyclotetrasiloxane, cyclopentasiloxane, and cyclohexasiloxane, and mixtures thereof. The carrier also may comprise, additionally or alternatively, nonvolatile silicones such as dimethicone and dimethicone copolyols, which have from about 2 to about 9 silicon atoms. Examples of suitable dimethicone and dimethicone copolyols include polyalkyl siloxanes, polyalkylaryl siloxanes, and polyether siloxane copolymers. In some preferred embodiments, the antiperspirant composition comprises from 32.0 to 50.0 wt. % cyclopentasiloxane.

The antiperspirant compositions, according to various embodiments, can be prepared by first combining the stearyl alcohol, the 14/16 fatty alcohol, and the polyethylene, if used, and then melting them or, alternatively, melting each component and then mixing them, to form a melted mixture. The remaining ingredients can be added to the melted mixture, either separately or as one or more premixtures, to form a liquid active mixture. The active mixture is then poured into canisters and permitted to cool at room temperature to form a soft solid, cream or gel antiperspirant composition.

The following is an exemplary embodiment of an antiperspirant composition, with each of the components set forth in weight percent of the antiperspirant composition. The example is provided for illustration purposes only and is not meant to limit the various embodiments of the antiperspirant composition in any way. All materials are set forth in weight percent.

| EXAMPLE 1 | |
|---|---|
| Ingredient | Weight percent |
| Cyclopentasiloxane | 32.0-50.0 |
| Aluminum zirconium Pentachlorohydrex GLY | 18.0-24.0 |
| Stearyl alcohol | 5.0-15.0 |
| Cetyl and/or Myristyl alcohol | 2.5-12.0 |
| Myristyl myristate | 1.0-8.0 |
| Castor wax | 3.0-10.0 |
| Polyethylene | 1.0-10.0 |
| PPG-14 butyl ether | 5.0-15.0 |
| Fragrance | 0.1-3.0 |
| Total | 100.00 |

Typically, these ingredients (except the fragrance) are mixed to form a homogeneous suspension, heating to about 75°-90°C to melt and dissolve the polyethylene, and cooling the mixture to form a stiff cream or soft solid, with the fragrance being added during cooling. This composition maintains its viscosity during storage.

Accordingly, various embodiments relating to a soft solid antiperspirant composition that comprises stearyl alcohol and cetyl and/or myristyl alcohol as desired structurants and avoid use of silicones have been provided. In this regard, the various embodiments comprise cetyl alcohol, myristyl alcohol, or a combination thereof. As noted above, while stearyl alcohol is a desired structurant in antiperspirant compositions because of its ability to impart structure to the compositions, in certain compositions it may cause crystals to form on the compositions after manufacture. It is believed that the cetyl alcohol, myristyl alcohol or a combination thereof, present in sufficient amounts in the antiperspirant composition, forms a fatty alcohol matrix with the stearyl alcohol that prevents the crystals from forming.

## Claims

1. A soft solid product, consisting of a container and a soft solid antiperspirant composition, wherein the soft solid antiperspirant composition comprises:
- an active antiperspirant compound, wherein the active antiperspirant compound is present in an amount of from 18.0 to 25.0 weight percent of the soft solid antiperspirant composition;
- a first fatty alcohol constituent comprising stearyl alcohol, wherein the first fatty alcohol is present in an amount of from 5.0 to 25.0 weight percent of the soft solid antiperspirant composition;
- a second fatty alcohol constituent comprising C₁₄ to C₁₆ fatty alcohol, wherein the second fatty alcohol is present in an amount of 2.5 to 12.0 weight percent of the soft solid antiperspirant composition; and
- wax having a melting point of at least 70°C, wherein the wax is present in an amount of from 3.0 to 10.0 weight percent of the soft solid antiperspirant composition,
and wherein the container is in the form of a canister 12, which includes
- a top covering 14 that is formed with a plurality of apertures 16 and
- a dial 18 threadably connected to a piston head 20, which is selectively raised, as the dial 18 is rotated,
by which the volume 22 between the piston head 20 and the top covering 14 is reduced and pressure is applied to the soft solid antiperspirant composition 10, resulting in the extrusion of a desired amount of the soft solid antiperspirant composition 10 from the canister 12 through the apertures 16.

2. A soft solid product according to claim 1, consisting of a container and a soft solid antiperspirant composition, wherein the soft solid antiperspirant composition consists of:
| Ingredient | Weight percent |
|---|---|
| Cyclopentasiloxane | 32.0-50.0 |
| Aluminum zirconium Pentachlorohydrex GLY | 18.0-24.0 |
| Stearyl alcohol | 5.0-15.0 |
| Cetyl and/or Myristyl alcohol | 2.5-12.0 |
| Myristyl myristate | 1.0-8.0 |
| Castor wax | 3.0-10.0 |
| Polyethylene | 1.0-10.0 |
| PPG-14 butyl ether | 5.0-15.0 |
| Fragrance | 0.1-3.0 |
| Total | 100.00 |
and wherein the container is in the form of a canister 12, which includes
- a top covering 14 that is formed with a plurality of apertures 16 and
- a dial 18 threadably connected to a piston head 20, which is selectively raised, as the dial 18 is rotated,
by which the volume 22 between the piston head 20 and the top covering 14 is reduced and pressure is applied to the soft solid antiperspirant composition 10, resulting in the extrusion of a desired amount of the soft solid antiperspirant composition 10 from the canister 12 through the apertures 16.

## Patentansprüche

1. Weiches Feststoffprodukt, das aus einem Behälter und einer weichen schweißhemmenden Feststoffzusammensetzung besteht, wobei die weiche schweißhemmende Feststoffzusammensetzung Folgendes umfasst:
- eine aktive schweißhemmende Verbindung, wobei die aktive schweißhemmende Verbindung in einer Menge von 18,0 bis 25,0 Gew.-% der weichen schweißhemmenden Feststoffzusammensetzung vorliegt;
- einen ersten Fettalkoholbestandteil, umfassend Stearylalkohol, wobei der erste Fettalkohol in einer Menge von 5,0 bis 25,0 Gew.-% der weichen schweißhemmenden Feststoffzusammensetzung vorliegt;
- einen zweiten Fettalkoholbestandteil, umfassend C₁₄- bis C₁₆-Fettalkohol, wobei der zweite Fettalkohol in einer Menge von 2,5 bis 12,0 Gew.-% der weichen schweißhemmenden Feststoffzusammensetzung vorliegt; und
- Wachs mit einem Schmelzpunkt von wenigstens 70 °C, wobei das Wachs in einer Menge von 3,0 bis 10,0 Gew.-% der weichen schweißhemmenden Feststoffzusammensetzung vorliegt;
und wobei der Behälter in der Form einer Büchse 12 ist, die Folgendes enthält:
- eine obere Abdeckung 14, die mit mehreren Öffnungen 16 ausgebildet ist, und
- eine Einstellscheibe 18, die durch ein Gewinde mit einem Kolbenkopf 20 verbunden ist, der beim Drehen der Einstellscheibe 18 selektiv angehoben wird,
wodurch das Volumen 22 zwischen dem Kolbenkopf 20 und der oberen Abdeckung 14 reduziert wird und Druck auf die weiche schweißhemmende Feststoffzusammensetzung 10 aufgebracht wird, was zum Extrudieren einer gewünschten Menge der weichen schweißhemmenden Feststoffzusammensetzung 10 aus der Büchse 12 durch die Öffnungen 16 führt.

2. Weiches Feststoffprodukt nach Anspruch 1, das aus einem Behälter und einer weichen schweißhemmenden Feststoffzusammensetzung besteht, wobei die weiche schweißhemmende Feststoffzusammensetzung aus Folgendem besteht:
| Inhaltsstoff | Gewichtsprozent |
|---|---|
| Cyclopentasiloxan | 32,0-50,0 |
| Aluminium-Zirconiumpentachlorhydrex GLY | 18,0-24,0 |
| Stearylalkohol | 5,0-15,0 |
| Cetyl- und/oder Myristylalkohol | 2,5-12,0 |
| Myristylmyristat | 1,0-8,0 |
| Rizinuswachs | 3,0-10,0 |
| Polyethylen | 1,0-10,0 |
| PPG-14-Butylether | 5,0-15,0 |
| Duftstoff | 0,1-3,0 |
| Gesamt | 100,00 |
und wobei der Behälter in der Form einer Büchse 12 ist, die Folgendes enthält:
- eine obere Abdeckung 14, die mit mehreren Öffnungen 16 ausgebildet ist, und
- eine Einstellscheibe 18, die durch ein Gewinde mit einem Kolbenkopf 20 verbunden ist, der beim Drehen der Einstellscheibe 18 selektiv angehoben wird,
wodurch das Volumen 22 zwischen dem Kolbenkopf 20 und der oberen Abdeckung 14 reduziert wird und Druck auf die weiche schweißhemmende Feststoffzusammensetzung 10 aufgebracht wird, was zum Extrudieren einer gewünschten Menge der weichen schweißhemmenden Feststoffzusammensetzung 10 aus der Büchse 12 durch die Öffnungen 16 führt.

## Revendications

1. Produit solide mou, constitué d'un récipient et d'une composition anti-transpirante solide molle, la composition anti-transpirante solide molle comprenant :
- un composé anti-transpirant actif, le composé anti-transpirant actif étant présent dans une quantité de 18,0 à 25,0 pourcent en poids de la composition anti-transpirante solide molle ;
- un premier constituant d'alcool gras comprenant l'alcool stéarylique, le premier alcool gras étant présent dans une quantité de 5,0 à 25,0 pourcent en poids de la composition anti-transpirante solide molle ;
- un second constituant d'alcool gras comprenant un alcool gras en C₁₄ à C₁₆, le second alcool gras étant présent dans une quantité de 2,5 à 12,0 pourcent en poids de la composition anti-transpirante solide molle ; et
- de la cire ayant un point de fusion d'au moins 70°C, la cire étant présente dans une quantité de 3,0 à 10,0 pourcent en poids de la composition anti-transpirante solide molle,
et le récipient se présentant sous la forme d'une cartouche 12, qui comprend
- un capot supérieur 14 qui est formé avec une pluralité d'orifices 16 et
- un disque ajouré 18 relié par vissage à une tête de piston 20 qui est soulevée de manière sélective lorsque le disque ajouré 18 est mis en rotation,
de sorte que le volume 22 entre la tête de piston 20 et le capot supérieur 14 est réduit et la pression est appliquée à la composition anti-transpirante solide molle 10, ce qui entraîne l'extrusion d'une quantité souhaitée de la composition anti-transpirante solide molle 10 de la cartouche 12 à travers les orifices 16.

2. Produit solide mou selon la revendication 1, constitué d'un récipient et d'une composition anti-transpirante solide molle, la composition anti-transpirante solide molle se composant de :
| Ingrédient | Pourcentage en poids |
|---|---|
| Cyclopentasiloxane | 32,0 à 50,0 |
| Pentachlorohydrex d'aluminum-zirconium GLY | 18,0 à 24,0 |
| Alcohol stéarylique | 5,0 à 15,0 |
| Alcohol cétylique et/ou myristylique | 2,5 à 12,0 |
| Myristate de myristyle | 1,0 à 8,0 |
| Huile de ricin hydrogénée | 3,0 à 10,0 |
| Polyéthylène | 1,0 à 10,0 |
| PPG-14 éther de butyle | 5,0 à 15,0 |
| Parfum | 0,1 à 3,0 |
| Total | 100,00 |
et le récipient se présentant sous la forme d'une cartouche 12, qui comprend
- un capot supérieur 14 qui est formé avec une pluralité d'orifices 16 et
- un disque ajouré 18 relié par vissage à une tête de piston 20 qui est soulevée de manière sélective lorsque le disque ajouré 18 est mis en rotation,
de sorte que le volume 22 entre la tête de piston 20 et le capot supérieur 14 est réduit et la pression est appliquée à la composition anti-transpirante solide molle 10, ce qui entraîne l'extrusion d'une quantité souhaitée de la composition anti-transpirante solide molle 10 de la cartouche 12 à travers les orifices 16.
